# EUROPEAN PATENT SPECIFICATION

(11) **EP 1 951 046 B1**
(45) Date of publication and mention of the grant of the patent: **18.07.2012**
(21) Application number: 06813613.4
(22) Date of filing: 21.08.2006
(51) Int. Cl.: A61K 31/395, A61K 31/255, A61K 31/675, A61K 31/4709, A61K 31/7068, A01N 43/00, C07D 255/02, A61P 35/02

(54) **METHODS TO ENHANCE CHEMOTHERAPY**
VERFAHREN ZUR VERBESSERUNG DER CHEMOTHERAPIE
METHODES PERMETTANT D'AUGMENTER L'EFFICACITE D'UNE CHIMIOTHERAPIE

(30) Priority: 19.08.2005 US 709978 P; 08.11.2005 US 734736 P
(43) Date of publication of application: 06.08.2008
(73) Proprietor: Genzyme Corporation, Cambridge, MA 02142 (US)
(72) Inventor: BRIDGER, Gary, J., Bellingham, WA 98225 (US)
(74) Representative: Duncan, Garreth Andrew
(86) International application number: PCT/US2006/032668
(87) International publication number: WO 2007/022523

(56) References cited:
- WO-A2-02/053138
- WO-A2-2005/082415
- WO-A2-2006/020891
- US-B1- 6 872 714
- JUAREZ J ET AL: "Effects of inhibitors of the chemokine receptor CXCR4 on acute lymphoblastic leukemia cells in vitro." LEUKEMIA (BASINGSTOKE), vol. 17, no. 7, July 2003 (2003-07), pages 1294-1300, XP002566663 ISSN: 0887-6924
- KURITZKES D. ET AL.: 'Novel Antiretroviral Agents' IMEDOPTIONS, LLC 01 November 2004, page 2, 14

## Description

### Related Applications

This application claims benefit of U.S. provisional applications Serial Numbers 60/709,978, filed 19 August 2005, and 60/734,736, filed 8 November 2005.

### Technical Field

The invention is in the field of treating myeloid leukemia. More particularly, the invention concerns compositions containing CXCR4 antagonists for use in treating such conditions.

### Background Art

A common approach to hematopoietic-related cancers, such as myeloid leukemias and lymphoid leukemias, is a session of chemotherapy to destroy the malignant cells combined with transplantation of hematopoietic progenitor cells either of autogeneic or allogeneic origin. It is believed that the lack of success often experienced with this treatment regimen is due to failure of the chemotherapy to completely eliminate the malignant hematopoietic cells or their precursors. The present invention improves this method by coupling it with administration of a compound that enhances the effect of chemotherapy with respect to these residual malignant or pre-malignant cells.

The compounds useful in the method of the invention are antagonists of the CXCR4 receptor that prevent its interaction with the cytokine stromal cell derived factor-1 (SDF-1). Many such agents are known in the art. Such agents are disclosed, for example, in U.S. Patent Nos. 5,021,409; 6,001,826; 5,583,131; 5,698,546; 5,817,807 and 6,506,770, and in PCT publications WO 92/16494; WO 93/12096; WO 95/18808; WO 00/02870; WO 00/56729; WO 01/44229; WO 02/22600; WO 02/22599; WO 02/34745, WO 03/055876; WO 04/091518 and WO 04/093217. WO 2004/087068 also describes CXCR4 antagonists and their use in the treatment or prevention of chemokine-related pathologies, including cancer.

We have previously found, and have disclosed in PCT publication WO 02/58653, that the certain CXCR4 antagonists, in particular, AMD3100, have the effect of increasing the white blood cell count. We have also found, and have disclosed in PCT publication WO 03/011277, that these antagonists have the effect of mobilizing progenitor cells and/or stem cells from the bone marrow to the circulating blood.

The chemokine receptor CXCR4 and its natural ligand SDF-1 appear to be important in the process of hematopoiesis (for reviews see Maekawa, T., et al., Internal Med. (2000) 39:90-100; Nagasawa, T., et al., Int. J. Hematol. (2000) 72:408-411). For example, CXCR4 or SDF-1 knock-out mice exhibit hematopoietic defects (Ma, Q., et al., Proc. Natl. Acad. Sci USA (1998) 95:9448-9453). It appears that SDF-1 is able to control the positioning and differentiation of cells bearing CXCR4 receptors whether these cells are stem cells (*i.e*., cells which are CD34+) or are progenitor cells (which result in formation of specified types of colonies in response to particular stimuli).

It appears that, within the microenvironment of the bone marrow, SDF-1 acts as a potent chemoattractant for immature and mature hematopoietic cells, and thus expression of CXCR4 on leukemic progenitor cells and leukemia cells may contribute to homing them to the bone marrow microenvironment. Elevated CXCR4 levels are detected on leukemic cells from patients with B chronic lymphocytic leukemia (B-CLL) (Mohle, R., et al., Leukemia (1999) 13:1954-1959). It further appears that autocrine secretion of SDF-1 by blood-derived adherent nurse-like cells in chronic lymphocytic leukemia (CLL) protects leukemic B cells from spontaneous apoptosis (Burger, J. A., et al., Blood (2000) 96:2655-2663). Enhanced levels were not detected on leukemic cells from patients with T-ALL or leukemic cells from patients with AML according to Mohle, *et al., supra;* Voermans, C., et al., Leukemia (2002) 16:650-657; Bradstock, K. F., et al., Leukemia (2000) 14:882-888; Dialynas, D. P., et al., Stem Cells (2001) 19:443-452; Shen, W., et al., Exp. Hematol. (2001) 29:1439-1447. However, it appears that expression levels of CXCR4 vary among various types of AML as reported by Rombouts, E. J., et al., Blood (2004) 104:550-557; Fukuda, S., et al., Blood (2005) 105:3117-3126. CXCR4 is also reported to mediate homing and engraftment of pre-B-ALL and AML cells to bone marrow, although other factors may be involved (Shen, *et al., supra;* Tavor, S., et al., Cancer Res. (2004) 64:2817-2824.). It was recently shown, in an *in vitro* context, that AMD3100 blocked SDF-1 induced chemotaxis of pre-B-ALL cells into bone marrow stroma layers, and enhanced the cytotoxic and antiproliferative effects of vincristine and dexamethasone (Juarez, J., et al., Leukemia (2003) 17:1294-1300.) These studies suggest that SDF-1/CXCR4 interactions are involved in the microenvironmental regulation of leukemic cells and such interaction plays a role in the resistance of residual, post-chemotherapy AML exposure to additional chemotherapeutic agents.

There is a need to mobilize pre-cancerous or cancerous cells from the bone marrow and into the peripheral blood system, where these cells can be exposed to chemotherapeutic agents. The current invention addresses such need by use of inhibitors of the CXCR4 receptor to potentiate the effects of standard chemotherapeutic agents, through the release and/or rapid movement of pre-leukemic cells and leukemic cells from the microenvironment of the bone marrow and into circulating blood prior to, or during, or after treatment by chemotherapy.

This invention may be used to treat subjects that may or may not require transplantation.

Citation of the above documents is not intended as an admission that any of the foregoing is pertinent prior art. All statements as to the date or representation as to the contents of these documents is based on the information available to the applicants and does not constitute any admission as to the correctness of the dates or contents of these documents, and not intended to be bound by any theory or hypothesis described in these documents.

### Disclosure of the Invention

The invention is directed to a comparison comprising as active ingredient a CXCR4 antagonist selected from 1,1'-[1,4-phenylene-bis(methylene)]-bis-1,4,8,11-tetraazacyclotetradecane or a pharmaceutically acceptable salt or metal complex thereof, for use in a method to treat a subject afflicted with a myeloid leukemia, where the treatment comprises mobilising myeloid leukemic cells from the bone marrow to the peripheral blood by administering said compositions to said subject, and administering one or more chemotherapeutic agents to said subject. The CXCR4 antagonist(s) may be administered before, during, and/or after chemotherapeutic regimens are administered.

In another aspect, the invention is directed to use of a composition as defined above in the manufacture of a medicament for treating myeloid leukemia, the treatment being as defined above.

The above CXCR4 antagonist useful in the invention is disclosed in U.S. Patent No. 5,583,1.31.

### Modes of Carrying Out the Invention

The invention employs compounds that inhibit the binding of SDF-1 to CXCR4 (CXCR4 antagonists). While not wishing to be bound by any theory, the compounds which inhibit the binding of SDF-1 to CXCR4 effect enhancement of chemotherapy by virtue of such inhibition, by depriving the malignant or pre-malignant cells from the protection of the stromal cells of the bone marrow.

As used herein, the term "pre-malignant cells" refers to cells that can form malignant hematopoietic or myeloid cells. The malignant hematopoietic or myeloid cells are those which characterize the conditions of myeloma, leukemia, and lymphoma. Particular forms of these diseases include acute myelitic leukemia (AML), acute lymphatic leukemia (ALL), multiple myeloma (MM), chronic myelogenous leukemia (CML), hairy cell leukemia (HCL), acute promyelocytic leukemia (APL), Chronic lymphocytic leukemia (CLL) and various lymphomas.

Chemotherapeutic compounds, or agents which may be used in the methods whose effectiveness is enhanced by the methods of the invention include carboplatin, carmustine, chlorambucil, dacarbazine, ifosfamide, lomustine, mechlorethamine, procarbazine, pentostatin, (2'deoxycoformycin), etoposide, teniposide, topotecan, vinblastine, vincristine, paclitaxel, dexamethasone, methylprednisolone, prednisone, all-trans retinoic acid, arsenic trioxide, interferon-alpha, rituximab (Rituxan^{®}), gemtuzumab ozogamicin, imatinib mesylate, cytarabine (cytosine arabinoside, Ara-C, Cytosar-U), melphalan, busulfan (Myleran^{®}), thiotepa, bleomycin, platinum (cisplatin), cyclophosphamide, Cytoxan^{®}, daunorubicin, doxorubicin, idarubicin, mitoxantrone, 5-azacytidine, cladribine, fludarabine, hydroxyurea, 6-mercaptopurine, methotrexate, 6-thioguanine, and many others.

A wide variety of chemotherapeutic methods are available in the art. The invention herein employs these standard methods or variations thereof but, in addition, provides for administration of the CXCR4 antagonists to enhance the effect of such methods. Preferably, these antagonists are administered prior to and/or concomitant with subjecting the patient to such methods. Administration may continue after the method has ceased as well, if desired. Dosage levels and mode of administration are interdependent. When given subcutaneously, for example, the dosage levels are in the range of 50 µg/kg - 1 mg/kg, preferably 200 µg/kg - 500 µg/kg. Dosage levels using oral administration may be higher and intravenous administration somewhat lower.

The CXCR4 antagonist used in the present invention is 1,1'-[1,4-phenylene-bis(methylene)]-bis-1,4,8,11-tetraazacyclotetradecane, set forth in U.S. Patent No. 5,583,131, and designated herein AMD3100, or a pharmaceutically acceptable salt or metal complex thereof.

As noted above, AMD3100 is an exemplary antagonist with the CXCR4 chemokine receptor (Gerlach, et al., J Biol. Chem. (2001) 276:14153-14160). This compound interferes with the binding of bone marrow stromal cell derived SDF-1 with CXCR4 on stem cells which leads to the release of hematopoietic stem cells from bone marrow into the circulation (Broxmeyer, et al., Blood (2001) 98:811a (Abstract)).

Compounds useful in the invention which are amines, may be administered or prepared in the forms of their acid addition salts or metal complexes thereof. Suitable acid addition salts include salts of inorganic acids that are biocompatible, including HCl, HBr, sulfuric, phosphoric and the like, as well as organic acids such as acetic, propionic, butyric and the like, as well as acids containing more than one carboxyl group, such as oxalic, glutaric, adipic and the like. Typically, at physiological pH, the compounds of the invention will be in the forms of the acid addition salts.

Compounds useful in the invention that are carboxylic acids or otherwise acidic may be administered or prepared in forms of salts formed from inorganic or organic bases that are physiologically compatible. Thus, these compounds may be prepared in the forms of their sodium, potassium, calcium, or magnesium salts as appropriate or may be salts with organic bases such as caffeine or ethylamine. These compounds also may be in the form of metal complexes.

When prepared as purified forms, the compounds may also be crystallized as the hydrates or other solvates. Those forms of the compounds used in the invention that contain chiral centers may be optically pure or may contain a mixture of stereoisomers, including racemic mixtures or mixtures of varying optical purity.

The CXCR4 antagonists may be formulated for administration to animal subject using commonly understood formulation techniques well known in the art. Formulations which are suitable for particular modes of administration and for compounds useful in the invention may be found in Remington's Pharmaceutical Sciences, latest edition, Mack Publishing Company, Easton, PA.

Preferably, the CXCR4 antagonists are administered by injection, most preferably by intravenous injection, but also by subcutaneous or intraperitoneal injection, and the like. Additional parenteral routes of administration include intramuscular and intraarticular injection. For intravenous or parenteral administration, the compounds are formulated in suitable liquid form with excipients as required. The compositions may contain liposomes or other suitable carriers. For injection intravenously, the solution is made isotonic using standard preparations such as Hank's solution.

Besides injection, other routes of administration may also be used. The compounds may be formulated into tablets, capsules, syrups, powders, or other suitable forms for administration orally. By using suitable excipients, these compounds may also be administered through the mucosa using suppositories or intranasal sprays. Transdermal administration can also be effected by using suitable penetrants and controlling the rate of release.

The formulation and route of administration chosen will be tailored to the individual subject, the nature of the condition to be treated in the subject, and generally, the judgment of the attending practitioner.

Suitable dosage ranges for the CXCR4 antagonists vary according to these considerations, but in general, the compounds are administered in the range of about 0.1 µg/kg-5 mg/kg of body weight; preferably the range is about 1 µg/kg-500 µg/kg up to 1 mg/kg of body weight. For a typical 70-kg human subject, thus, the dosage range is from about 0.7 µg-350 mg. Dosages may be higher when the compounds are administered orally or transdermally as compared to, for example, i.v. administration.

The CXCR4 antagonists may be administered as a single bolus dose, a dose over time, as in i.v. or transdermal administration, or in multiple dosages. The CXCR4 antagonists may be administered along with other factors that aid in mobilization, or other factors that are nutritional or therapeutically beneficial. The additional factor(s) may be administered in the same composition, in different compositions but simultaneously, or in a tandem protocol with the administration of the CXCR4 antagonist. Among additional factors that can be included are recombinant G-CSF (Neupogen^{®}, Granocyte^{®}/Neutrogin^{®}, and Stemgen^{®}), a covalent conjugate of recombinant G-CSF (Neulasta^{®}), granulocyte-macrophage colony stimulating factor (GM-CSF) (such as Leukine^{®}, and Leucomax^{®}), Interleukin-1 (IL-1), Interleukin-3 (IL-3), Interleukin-8 (IL-8), PIXY-321 (GM-CSF/IL-3 fusion protein), macrophage inflammatory protein, stem cell factor, and thrombopoietin, as well as antibiotics, vitamins, herbal extracts, anti-inflammatories, nutrients, antipyretics, analgesics, cyclophosphamide and the like.

As noted, the compounds as administered in conjunction with chemotherapeutic methods. These methods are those generally employed in the treatment of myeloid leukemia that are subject to treatment using the composition of the invention. A wide variety of such methods is known in the art.

Subjects that will respond favorably to the composition used in the invention include medical and veterinary subjects generally, including human patients. Among other subjects for whom the composition used in the invention is useful are cats, dogs, large animals, avians such as chickens, and the like, other than standard research rodents such as laboratory mice, rabbits, or rats. In general, any subject that exhibits a hematopoietic or myelitic malignancy would benefit from the composition used in the invention.

A wide variety of chemotherapeutic protocols is employed, many of such protocols involving combinations of drugs administered simultaneously or in tandem. The CXCR4 antagonists may be administered at various points in the simultaneous or tandem protocols. For example, one protocol for AML involves combinations of busulfan and fludarabine. These drugs are administered intravenously. The CXCR4 antagonist may be administered several hours before the first administration of this drug which is repeated over several days. The CXCR4 antagonist may be administered each day prior to or during the administration of the fludarabine, or only typical, busulfan is administered subsequent to fludarabine over several days, and the CXCR4 antagonist may be administered each day along with, before, or after the administration of the busulfan, one administration before, during or after treatment may be required.

Various combinations of the foregoing agents are used in such protocols, and the timing and frequency of CXCR4 administration is subject to routine optimization, well within ordinary skill.

Having now generally described the invention, the same will be more readily understood through reference to the following examples which are provided by way of illustration, and are not intended to be limiting of the present invention, unless specified.

### Example 1

This example describes a murine model of human acute promyelocytic leukemia (APL) used to determine the effect of a CXCR4 antagonist on the mobilization of APL cells into the peripheral blood, and on their sensitivity to chemotherapeutic agents known to affect the proliferation of these cells. Murine APL cells were generated by "knocking-in" the PML-RARα cDNA from human APL into the murine cathepsin G locus (Westervelt, et al., PubMed(2003) 102(5):1857-1865) that resulted in the overexpression in the murine promyelocyte compartment. Mouse APL cells, after injection into syngeneic recipients, home preferentially to the bone marrow microenvironment in a manner similar to what is observed in human AML, and expands there for 20-30 days after circulating in high numbers in the peripheral blood. This ultimately leads to death of the animals by 50 to 80 plus days.

Using a murine model of APL, one may determine whether leukemic cells are "mobilized" in a similar manner to normal stem cells after treatment with AMD3100. In one instance, AMD3100 (5 mg/kg) injected immediately at the same time when APL cells were injected did not have any impact on the engraftment (short or long term) of either normal bone marrow stem cells or the leukemic cells. However, where AMD3100 was administered 11 days after APL injection, a rapid mobilization of the leukemic cells was observed. Forty percent (2/5) of mice that received a single dose of AMD3100 on day +11 after APL injection died 2 to 4 hours after the administration of AMD3100. It was observed that administration of AMD3100 on day +11 induced a 3-fold increase in total white blood cell (WBC) counts, and a 10-fold increase in the leukemic blasts into peripheral blood.

When AMD3100 was administered concomitantly with cytarabine (200 mg/kg) on day +11 into mice, this treatment significantly prolonged the overall survival of mice, compared with mice treated only with cytarabine. Based on the observed results, it may be possible that tumor resistance may be overcome by potentiating the effects of chemotherapeutic agents by mobilizing tumor cells from the bone marrow into the peripheral blood.

### Example 2

### Clinical Study

The *in vivo* effect of the CXCR4 antagonist AMD3100 was studied in three patients with AML, who had insufficient mobilization of CD34+ cells for autologous stem cell transplantation with G-CSF and/or Cytoxan^{®}. The combination of G-CSF and AMD3100 (for 3-4 days) resulted in massive mobilization of leukemic cells into the circulation in a time-dependent fashion, as determined by flow cytometry and interphase FISH analysis of their respective cytogenetic abnormalities.

| Patient# | Cytogenetics, FCM | % (+) cells Day 2 | % (+) cells Day 4/5 | Apheresis CD34x10⁶/kg |
|---|---|---|---|---|
| 1 | Trisomy 21 | | 22.6 | 57.0 |
| | FCM CD7/33 | | 22.0 | |
| 2 | Trisomy 9 | 28.6 | 68.6 | 4.8 |
| | Inv 16 | 29.0 | 75.8 | |
| | FCM CD 13/33 | | 74.0 | |
| 3 | Mono 17 | 40.4 | 53.4 | 8.7 |
| | 5q31 | 37.5 | 49.6 | |
| | FCM CD 13/33 | | 50.0 | |

It is understood that the foregoing detailed description and accompanying examples are merely illustrative, and are not to be taken as limitations upon the scope of the invention.

## Claims

1. A composition comprising as active ingredient a CXCR4 antagonist selected from 1,1'-[1,4-phenylene-bis-(methylene)]-bis-1,4,8,11-tetxaazacyclotettradecane or a pharmaceutically acceptable salt or metal complex thereof, for use in a method of treatment a myeloid leukemia in a subject afflicted with a myeloid leukemia, where the treatment comprises:
mobilizing myeloid leukemic cells from the bone marrow to the peripheral blood by administering said composition to said subject, and
administering at least one chemotherapeutic agent to said subject,

2. The composition of claim 1 wherein the active ingredient is administered to said subject in the dosage range of about 0.1 µg/kg-5 mg/kg of body weight.

3. The composition of claim 1 wherein the active ingredient is 1,1'-[1,4-phenylene-bis-(methylene)]-bis-1,4,8,11-tetfaazacyclotetradecane.

4. The composition of any of claims 1-3 wherein the chemotherapeutic agent is selected from the group consisting of carboplatin, carmustine, chlorambucil, dacarbazine, ifosfamide, lomustine, mechlorethamine, procarbazine, pentostatin, 2'-deoxycoformycin, etoposide, teniposide, topotecan, vinblastine, vincristine, paclitaxel, dexamethasone, methylprednisolone, prednisone, all-trans retinoic acid, arsenic trioxide, interferon-alpha, rituximab, gemtuzumab, ozogamicin, imatinib mesylate, cytarabine, melphalan, busulfan, thiotepa, bleomycin, cisplatin, cyclophosphamide, daunorubicin, doxorubicin, idarubicin, mitoxantrone, 5-azacytidine, cladribine, fludarabine, hydroxyurea, 6-mereaptopurine, methotrexate, and 6-thioguanine.

5. The composition of claim 4, wherein the chemotherapeutic agent is mitoxantrone, etoposide or cytarabine, or a combination thereof.

6. The composition of claim 4, wherein the chemotherapeutic agent is cytarabine,

7. The composition of claim 4, wherein the chemotherapeutic agent is daunorubicin, cytarabine or a combination thereof.

8. The composition of claim 4, wherein the chemotherapeutic agent is fludarabine, cytarabine or a combination thereof.

9. The composition of claim 4, wherein the chemotherapeutic agent is fludarabine, cytarabine, idarubicin or a combination thereof,

10. The composition of any one of claims 1-9 further comprising G-CSF.

11. The composition of any one of claims 1-10 wherein the myeloid leukemia is acute myeloid leukemia.

12. The composition of any one of claims 1-10 wherein the myeloid leukemia is chronic myeloid leukemia.

13. Use of a composition as defined in any one of claims 1-12 in the manufacture of a medicament for treating myeloid leukemia in a subject afflicted with acute myeloid leukemia, wherein the treatment comprises
mobilizing leukemic cells from the bone marrow to the peripheral blood by administering said composition to said subject, and
administering at least one chemotherapeutic agent to said subject.

## Patentansprüche

1. Zusammensetzung, die als einen aktiven Bestandteil einen CXCR4-Antagonisten umfasst, der ausgewählt ist unter 1,1'-[1,4-Phenylen-bis-(methylen)]-bis-1,4,8,11-tetraazacyclotetradecan oder einem pharmazeutisch verträglichen Salz oder Metallkomplex davon, für die Verwendung bei einem Verfahren zum Behandeln einer myeloischen Leukämie in einem Patienten, der unter einer myeloischen Leukämie leidet, wobei die Behandlung umfasst:
Mobilisierung von myeloischen Leukämiezellen aus dem Knochenmark in das periphere Blut durch Verabreichen der Zusammensetzung an den Patienten und
Verabreichung von wenigstens einem chemotherapeutischen Mittel an den Patienten.

2. Zusammensetzung nach Anspruch 1, wobei der aktive Bestandteil an den Patienten in einem Dosierungsbereich von etwa 0,1 µg/kg-5 mg/kg Körpergewicht verabreicht wird.

3. Zusammensetzung nach Anspruch 1, wobei der aktive Bestandteil 1,1'-[1,4-Phenylen-bis-(methylen)]-bis-1,4,8,11-tetraazacyclotetradecan ist.

4. Zusammensetzung nach einem der Ansprüche 1 bis 3, wobei das chemotherapeutische Mittel aus der Gruppe ausgewählt ist, die aus dem Folgenden besteht: Carboplatin, Carmustin, Chlorambucil, Dacarbazin, Ifosfamid, Lomustin, Mechlorethamin, Procarbazin, Pentostatin, 2'-Desoxycoformycin, Etoposid, Teniposid, Topotecan, Vinblastin, Vincristin, Paclitaxel, Dexamethason, Methylprednisolon, Prednison, all-trans-Retinsäure, Arsentrioxid, Interferon-alpha, Rituximab, Gemtuzumab, Ozogamicin, Imatinibmesylat, Cytarabin, Melphalan, Busulfan, Thiotepa, Bleomycin, Cisplatin, Cyclophosphamid, Daunorubicin, Doxorubicin, Idarubicin, Mitoxantron, 5-Azacytidin, Cladribin, Fludarabin, Hydroxyharnstoff, 6-Mercaptopurin, Methotrexat und 6-Thioguanin.

5. Zusammensetzung nach Anspruch 4, wobei das chemotherapeutische Mittel Mitoxantron, Etoposid oder Cytarabin oder eine Kombination davon ist.

6. Zusammensetzung nach Anspruch 4, wobei das chemotherapeutische Mittel Cytarabin ist.

7. Zusammensetzung nach Anspruch 4, wobei das chemotherapeutische Mittel Daunorubicin, Cytarabin oder eine Kombination davon ist.

8. Zusammensetzung nach Anspruch 4, wobei das chemotherapeutische Mittel Fludarabin, Cytarabin oder eine Kombination davon ist.

9. Zusammensetzung nach Anspruch 4, wobei das chemotherapeutische Mittel Fludarabin, Cytarabin, Idarubicin oder eine Kombination davon ist.

10. Zusammensetzung nach einem der Ansprüche 1 bis 9, welche weiterhin G-CSF umfasst.

11. Zusammensetzung nach einem der Ansprüche 1 bis 10, wobei die myeloische Leukämie akute myeloische Leukämie ist.

12. Zusammensetzung nach einem der Ansprüche 1 bis 10, wobei die myeloische Leukämie chronische myeloische Leukämie ist.

13. Verwendung einer Zusammensetzung, wie sie in einem der Ansprüche 1 bis 12 definiert ist, zur Herstellung eines Arzneimittels für die Behandlung von myeloischer Leukämie in einem Patienten, der unter akuter myeloischer Leukämie leidet, wobei die Behandlung umfasst
Mobilisierung von Leukämiezellen aus dem Knochenmark in das periphere Blut durch Verabreichung der Zusammensetzung an den Patienten und
Verabreichung von wenigstens einem chemotherapeutischen Mittel an den Patienten

## Revendications

1. Composition comprenant, comme ingrédient actif, un antagoniste CXCR4 choisi parmi le 1,1'-[1,4-phénylène-bis-(méthylène)]-bis-1,4,8,11-tétraazacyclotétradécane ou son sel ou complexe métallique pharmaceutiquement acceptable, pour son utilisation dans une méthode de traitement d'une leucémie myéloïde chez un sujet souffrant d'une leucémie myéloïde, dans laquelle le traitement comprend :
la mobilisation des cellules leucémiques myéloïdes de la moelle osseuse vers le sang périphérique en administrant ladite composition audit sujet, et
l'administration d'au moins un agent chimiothérapeutique audit sujet.

2. Composition selon la revendication 1, dans laquelle l'ingrédient actif est administré audit sujet, dans une gamme de dosage d'environ 0,1 µg/kg-5 mg/kg de masse corporelle.

3. Composition selon la revendication 1, dans laquelle l'ingrédient est le 1,1'-[1,4-phénylène-bis-(méthylène)]-bis-1,4,8,11-tétraazacyclotétradécane.

4. Composition selon l'une quelconque des revendications 1-3, dans laquelle l'agent chimiothérapeutique est choisi dans le groupe constitué de carboplatine, de carmustine, de chlorambucil, de dacarbazine, d'ifosfamide, de lomustine, de méchloréthamine, de procarbazine, de pentostatine, de 2'-déoxycoformycine, d'étoposide, de téniposide, de topotécan, de vinblastine, de vincristine, de paclitaxel, de dexaméthasone, de méthylprednisolone, de prednisone, d'acide tout-trans rétinoïque, de trioxyde d'arsenic, d'interféron alpha, de rituximab, de gemtuzumab, d'ozogamicine, de mésylate d'imatinib, de cytarabine, de melphalan, de busulfan, de thiotépa, de bléomycine, de cisplatine, de cyclophosphamide, de daunorubicine, de doxorubicine, d'idarubicine, de mitoxantrone, de 5-azacytidine, de cladribine, de fludarabine, d'hydroxy-urée, de 6-mercaptopurine, de méthotrexate, et de 6-thioguanine.

5. Composition selon la revendication 4, dans laquelle l'agent chimiothérapeutique est la mitoxantrone, l'étoposide ou la cytarabine ou une combinaison de ceux-ci.

6. Composition selon la revendication 4, dans laquelle l'agent chimiothérapeutique est la cytarabine.

7. Composition selon la revendication 4, dans laquelle l'agent chimiothérapeutique est la daunorubicine, la cytarabine ou une combinaison de ceux-ci.

8. Composition selon la revendication 4, dans laquelle l'agent chimiothérapeutique est la fludarabine, la cytarabine ou une combinaison de ceux-ci.

9. Composition selon la revendication 4, dans laquelle l'agent chimiothérapeutique est la fludarabine, la cytarabine, l'idarubicine ou une combinaison de ceux-ci.

10. Composition selon l'une quelconque des revendications 1-9, comprenant en outre du G-CSF.

11. Composition selon l'une quelconque des revendications 1-10, dans laquelle la leucémie myéloïde est une leucémie myéloïde aiguë.

12. Composition selon l'une quelconque des revendications 1-10, dans laquelle la leucémie myéloïde est une leucémie myéloïde chronique.

13. Utilisation d'une composition telle que définie dans l'une quelconque des revendications 1-12, dans la fabrication d'un médicament pour traiter la leucémie myéloïde chez un sujet souffrant d'une leucémie myéloïde aiguë, dans laquelle le traitement comprend
la mobilisation des cellules leucémiques de la moelle osseuse vers le sang périphérique en administrant ladite composition audit sujet et
l'administration d'au moins un agent chimiothérapeutique audit sujet.
